# FASCICULE DE BREVET EUROPEEN

(11) **EP 1 230 947 B1**
(45) Date de publication et mention de la délivrance du brevet: **03.09.2003**
(21) Numéro de dépôt: 02290310.8
(22) Date de dépôt: 08.02.2002
(51) Int. Cl.: A61N 1/37

(54) **Dispositif médical implantable actif, notamment stimulateur cardiaque, défibrillateur et/ou cardioverteur ou dispositif multisite, avec test de capture cycle à cycle**
Aktive implantierbare medizinische Vorrichtung, insbesondere Herzschrittmacher, Defibrillator, und/oder Kardiovertierer oder Mehrstelleneinrichtung, mit Schlag-zu-Schlag Einfangstest
Active implantable medical device, in particular pacemaker, defibrillator and/or cardioverter or multisite device, with beat-to-beat capture assay

(30) Priorité: 09.02.2001 FR 0101797
(43) Date de publication de la demande: 14.08.2002
(73) Titulaire: ELA MEDICAL, 92541 Montrouge (FR)
(72) Inventeur: Pons, Pascal, 38920 Crolles (FR); Dal Molin, Renzo, 92320 Chatillon (FR)
(74) Mandataire: Dupuis-Latour, Dominique

(56) Documents cités:
- EP-A- 0 813 889
- WO-A-93/02741
- US-A- 5 591 211
- US-A- 6 002 962
- US-B1- 6 169 921

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement l'ajustement de l'amplitude (niveau de tension) des impulsions de stimulation au cours du temps.

Le niveau de stimulation des cavités cardiaques (ventriculaires ou auriculaires) est une valeur typiquement comprise entre 1,5 et 7,5 V, ajustable par pas de 0,5 V. Cette amplitude doit bien entendu être suffisamment élevée pour provoquer la dépolarisation de la cavité myocardique ; il faut cependant éviter des valeurs trop élevées pour ménager la durée de vie de la pile, car l'énergie de stimulation appliquée au myocarde, et donc la consommation correspondante du dispositif, est proportionnelle au carré de l'amplitude (et également à la durée) de l'impulsion.

Le test du seuil d'efficacité de la stimulation ou "test de capture" peut être effectué à intervalles réguliers, par exemple toutes les six heures, par mise en oeuvre d'un algorithme de test automatique décrit notamment dans le WO-A-93/02741 (Ela Médical). L'amplitude des impulsions de stimulation est ensuite ajustée sur la base du seuil de capture ainsi mesuré, avec une marge de sécurité importante : le niveau ajusté est typiquement le double de la valeur du seuil de capture, entre un minimum (typiquement 1,5 V) et un maximum (typiquement 5 V).

Une autre technique - à laquelle se rapporte la présente invention - consiste à opérer un test de capture "cycle à cycle", c'est-à-dire à examiner à chaque cycle cardiaque si la stimulation a été efficace ou non.

À cet effet, la tension de stimulation est maintenue proche de la tension pour laquelle il n'y a plus de capture. En cas d'élévation du seuil de capture du patient, l'absence de capture est détectée dans les 63 ms (typiquement) qui suivent la stimulation inefficace.

Cette détection de perte de capture entraîne deux conséquences :
- à la prochaine stimulation, le niveau de la tension de stimulation sera accrue d'un pas, pour compenser l'élévation du seuil de capture du patient,
- mais surtout, une contre-stimulation à énergie plus importante doit être appliquée immédiatement (c'est-à-dire au bout des 63 ms suivant la stimulation inefficace), afin de compenser sans attendre l'absence de dépolarisation du myocarde.

Cette technique est particulièrement avantageuse, car elle permet de s'affranchir de la marge de sécurité importante qui était retenue lorsque le seuil de capture était mesuré seulement à intervalles périodiques, par exemple toutes les six heures. La durée de vie de la pile peut ainsi être allongée de façon substantielle.

Toutefois, la nécessité de délivrer une contre-stimulation beaucoup plus fréquemment peut perturber le fonctionnement du stimulateur et induire une surconsommation risquant de réduire, finalement, les avantages escomptés par la mise en oeuvre de cette technique de capture cycle à cycle.

De plus, il est indispensable de ne pas retarder la contre-stimulation, car un tel retard créerait un risque physiologique pour le patient.

L'un des buts de la présente invention est de proposer une nouvelle configuration des circuits de stimulation permettant d'éviter ces risques lors de la mise en oeuvre d'une technique où la capture est testée cycle à cycle, c'est-à-dire où la tension de stimulation est maintenue proche de la tension pour laquelle il n'y a plus de capture.

A cet effet, la présente invention propose un dispositif médical implantable actif du type générique précité, c'est-à-dire comportant au moins un étage de stimulation à faible énergie d'une cavité cardiaque avec : un condensateur de sortie ; un circuit de charge de ce condensateur à une tension de stimulation prédéterminée, proche du seuil d'efficacité de stimulation du patient porteur du dispositif ; des premiers moyens commutateurs, aptes à relier ce condensateur à une borne de stimulation du dispositif, elle-même reliée à une électrode de stimulation ; des moyens de test de capture, aptes à déterminer, après délivrance d'une stimulation, si celle-ci a été efficace ou si, au contraire, il y a eu perte de capture ; des moyens de réajustement de la tension de stimulation en fonction du résultat du test de capture ; et des moyens de délivrance d'une contre-stimulation après test de capture si le résultat de ce test révèle une perte de capture.

Selon l'invention, les moyens de délivrance d'une contre-stimulation incluent des moyens condensateurs supplémentaires et des seconds moyens commutateurs, aptes à relier ces moyens condensateurs supplémentaires à ladite borne de stimulation du dispositif.

Dans un premier mode de réalisation, les moyens condensateurs supplémentaires comprennent un condensateur spécifique, distinct dudit condensateur de sortie, et le circuit de charge est également apte à charger ce condensateur spécifique à une tension de contre-stimulation supérieure à ladite tension de stimulation.

Dans un second mode de réalisation, les moyens condensateurs supplémentaires incluent un condensateur additionnel, le circuit de charge est également apte à charger séparément ce condensateur additionnel, et les seconds moyens commutateurs sont des moyens aptes à relier en série le condensateur de sortie et le condensateur additionnel pendant la délivrance de la contre-stimulation.

Dans l'un ou l'autre cas, s'il est prévu au moins deux étages de stimulation distincts, ledit condensateur spécifique ou additionnel peut être avantageusement un condensateur de sortie appartenant à un second étage autre que ledit étage de stimulation, par exemple des étages respectifs auriculaire/ventriculaire droit, auriculaire/ventriculaire gauche, ventriculaire droit/ventriculaire gauche, ou inversement.

Le second mode de réalisation peut en particulier être obtenu par une configuration dans laquelle :
- une première armature du condensateur de sortie est reliée à la fois à une première sortie du circuit de charge et à un premier commutateur relié par ailleurs sélectivement à une première borne de stimulation,
- la seconde armature du condensateur de sortie est reliée à un deuxième commutateur relié par ailleurs sélectivement à la masse,
- une première armature du condensateur additionnel est reliée à la fois à une seconde sortie du circuit de charge et à un troisième commutateur relié par ailleurs sélectivement à une seconde borne de stimulation, et
- un quatrième commutateur relie sélectivement la seconde armature du condensateur de sortie et la première armature du condensateur additionnel.

L'invention sera mieux comprise à la lecture de la description détaillée ci-dessous, faite en référence aux dessins annexés.
La figure 1 illustre un premier mode de réalisation de l'invention.
La figure 2 illustre un second mode de réalisation de l'invention.

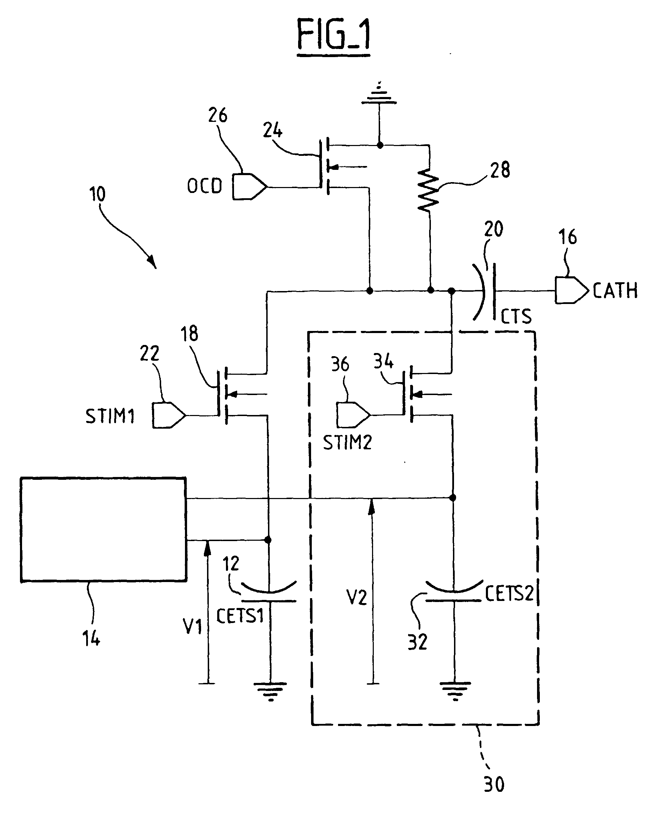

Sur la figure 1, la référence 10 désigne l'étage de sortie d'un générateur de stimulateur cardiaque.

L'impulsion de stimulation est obtenue par décharge d'un condensateur CETS1, référencé 12, amené à une tension de charge V1 par un circuit de charge 14. Ce circuit de charge 14 est généralement constitué d'un multiplicateur de tension qui peut générer une tension continue multiple de la tension de la pile d'alimentation (non représentée) du stimulateur. La charge est régulée afin d'être arrêtée quand la tension programmée V1 est atteinte. La tension V1 est généralement comprise entre 1,0 et 7,5 V, ajustable par pas de 0,5 V. La capacité du condensateur 12 est généralement de 10 µF.

L'énergie accumulée dans le condensateur 12 est transférée vers une borne de sortie CATH, référencée 16, du stimulateur, par exemple reliée à l'électrode distale d'une sonde de stimulation auriculaire ou ventriculaire. La décharge est commandée par la mise en conduction d'un transistor 18 dont la grille est commandée par la borne STIM1, référencée 22, via un condensateur de liaison CTS, référencé 20, typiquement de capacité 10 µF. Ce condensateur 20, outre sa fonction consistant à empêcher la création d'un courant continu, a aussi pour rôle de protéger le patient contre un éventuel courant continu en cas de défaillance des transistors de stimulation, comme le transistor 18.

Après stimulation, le condensateur 20 (qui s'est chargé pendant la stimulation) est déchargé par mise à la masse en rendant conducteur le transistor 24 dont la grille est commandée par la borne OCD (*Output Capacitor Discharge*), référencée 26. La résistance 28, typiquement de 20 kΩ, permet d'achever la décharge du condensateur 20 après l'ouverture du transistor 24, et également d'assurer une compensation des courants de fuite dans le circuit.

Avec cette seule configuration, si l'on fonctionnait en "capture cycle à cycle", c'est-à-dire avec une tension de stimulation proche de la tension pour laquelle il n'y a plus de capture, pour déclencher une contre-stimulation à énergie plus importante il serait nécessaire de recharger le condensateur 12 pendant les 63 ms suivant la stimulation, et donc opérer cette charge à chaque cycle, par sécurité : en effet, il n'est pas souhaitable de retarder la contre-stimulation pour permettre un complément de charge du condensateur 12 après détection d'une stimulation inefficace, car ceci perturberait le fonctionnement du stimulateur.

L'invention propose de compléter cette configuration, en elle-même connue, par le circuit compris dans le cadre en tiretés 30, afin de pouvoir, en cas de perte de capture, appliquer immédiatement une contre-stimulation à énergie plus importante et sans surconsommation inacceptable.

Plus précisément, l'invention propose de rajouter un condensateur CETS2, référencé 32, chargé en permanence à une tension supérieure à celle, V1, du condensateur 12. Cette tension de charge V2 est avantageusement délivrée par le même circuit 14 que celui servant à produire la tension V1 de charge du condensateur 12.

Ce condensateur 32 sert seulement à délivrer la contre-stimulation et, tant que la capture est efficace, il est simplement maintenu à sa tension de charge nominale V2, n'entraînant ainsi qu'une surconsommation négligeable (courant de fuite).

En revanche, en cas de perte de capture, le condensateur 32 est déchargé vers la borne 16 pour assurer la contre-stimulation, par fermeture du transistor 34 commandée par la borne STIM2, référencée 36. Le condensateur 32, étant chargé en permanence, est immédiatement prêt à assurer son rôle, de sorte que la contre-stimulation pourra être appliquée sans délai à la fin de l'intervalle de détection de l'absence de capture.

Comme le condensateur 12, le condensateur 32 de contre-stimulation est déchargée dans la borne 16 via le condensateur de liaison 20, qui sera déchargé par le transistor 24 et la résistance 28 après la contre-stimulation, de la même manière qu'après une stimulation normale.

Dans le premier mode de réalisation que l'on vient de décrire en référence à la figure 1, le condensateur de contre-stimulation 32 est un condensateur dédié, spécifique à cette fonction.

Dans un but notamment de réduction des coûts, le rôle de ce condensateur dédié peut être joué par un condensateur existant au sein du stimulateur, dès lors que la tension aux bornes y est supérieure à celle du condensateur de stimulation 12.

Ce condensateur existant peut être notamment celui d'un autre étage de stimulation, tel que l'étage de stimulation ventriculaire gauche, de l'étage de stimulation auriculaire ou de tout autre étage de stimulation disponible, tout particulièrement dans les dispositifs multisite, dès lors que ce condensateur ne doit pas être utilisé simultanément à d'autres fins.

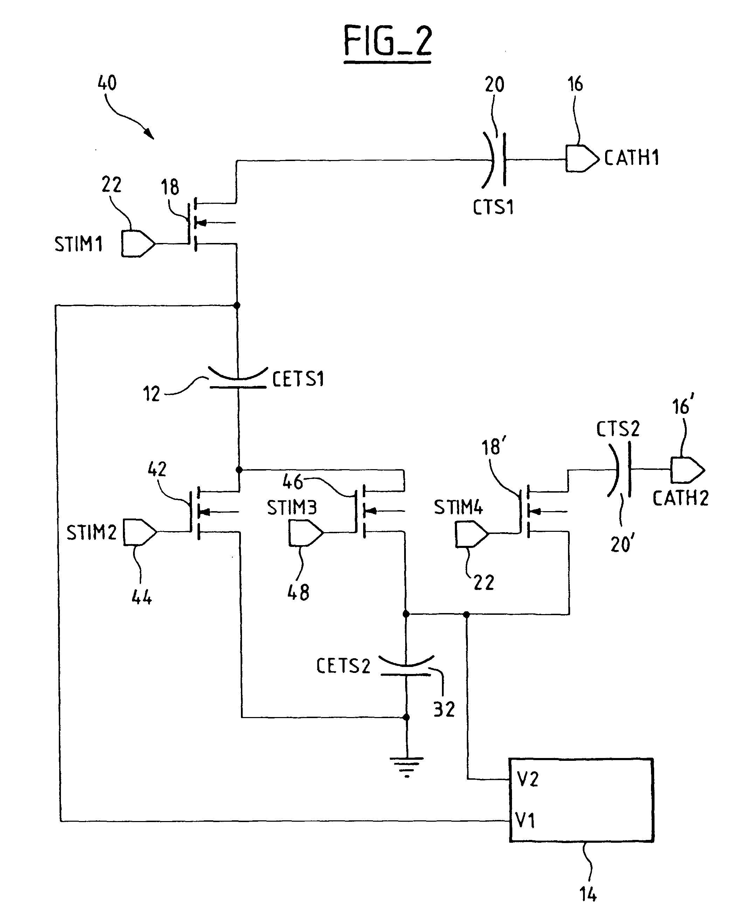

La figure 2 illustre un autre mode de réalisation, selon ce principe.

L'étage de stimulation double 40 comporte deux condensateurs de stimulation CETS1 et CETS2, référencés respectivement 12 et 32, chargés de la manière que l'on indiquera plus bas par un circuit de charge 14 susceptible de générer des tensions correspondantes V1 et V2.

La décharge peut être opérée sur deux bornes de sortie CATH1 et CATH2, référencées respectivement 16 et 16'.

La décharge sur la borne 16 est opérée par fermeture des transistors 18 et 42 (pour amener à la masse l'armature opposée du condensateur 12), commandés par application de signaux de commande sur les bornes correspondantes STIM1 et STIM2, référencées 22 et 44. La décharge sur la borne 16' est opérée par fermeture du transistor 18', commandé par application d'un signal de commande sur la borne correspondante STIM4, référencée 22'.

Les courants de décharge sont appliqués aux bornes 16 et 16' via des condensateurs de liaison CTS1 et CTS2, référencés 20 et 20' (par simplification, les transistors et résistances permettant de gérer la décharge des condensateurs de liaison 20 et 20' après stimulation ou contre-stimulation n'ont pas été représentés - ces composants sont identiques aux composants référencés 24, 26 et 28 de la figure 1).

La borne 16 étant reliée à l'électrode de stimulation du ventricule droit, la borne 16' est par exemple reliée à l'électrode de stimulation du ventricule gauche, ou d'une oreillette.

Des transistors 42 et 46, commandés par des bornes respectives STIM2 et STIM3, référencées 44 et 48, permettent de relier ensemble les condensateurs 12 et 32 de manière à les utiliser soit isolément, soit en série pour additionner les tensions à leurs bornes.

Le transistor 42 est monté entre l'une des armatures du condensateur 12 (opposée à celle reliée au transistor de décharge 18) et la masse.

Le transistor 46 est monté entre cette même armature du condensateur 12 et l'armature du condensateur 32 reliée au transistor de décharge 18', l'autre armature du condensateur 32 étant reliée directement à la masse.

Les transistors 18, 18', 42 et 46 sont commandés de la manière suivante selon que l'on souhaite charger l'un ou l'autre des condensateurs, ou bien décharger l'un ou l'autre des condensateurs, ou encore appliquer une contre-stimulation (dans l'explication qui suit on suppose, lorsque cela n'est pas précisé, que les transistors sont tous à l'état bloquant, c'est-à-dire en circuit ouvert) :
1) charge du condensateur 12 par le circuit 14 à la tension V1 : fermeture (mise en conduction) du transistor 42 ;
2) charge du condensateur 32 par le circuit 14 à la tension V2 : tous les transistors (notamment le transistor 46) restent ouverts ;
3) stimulation à tension V1 sur la borne 16 : fermeture des transistors 18 et 42 (le transistor 46 restant ouvert) de manière à permettre la décharge du condensateur 12 chargé à la tension V1 ;
4) application d'une contre-stimulation sur perte de capture : fermeture des transistors 18 et 46 (le transistor 42 restant ouvert), ce qui a pour effet de mettre en série les condensateurs 12 et 32 et ainsi d'appliquer à la borne 16 les tensions cumulées de V1 et V2, grâce à l'énergie additionnelle accumulée à la tension V2 dans le condensateur 32 ;
5) stimulation à tension V2 sur la borne 16' : fermeture du transistor 18' (le transistor 46 restant ouvert) de manière à permettre la décharge du condensateur 32 chargé à la tension V2.

## Revendications

1. Un dispositif médical implantable actif, notamment un stimulateur cardiaque, défibrillateur et/ou cardioverteur ou dispositif multisite, comportant au moins un étage de stimulation à faible énergie d'une cavité cardiaque, cet étage de stimulation comprenant :
- un condensateur de sortie (12),
- un circuit (14) de charge de ce condensateur à une tension (V1) de stimulation prédéterminée, proche du seuil d'efficacité de stimulation du patient porteur du dispositif,
- des premiers moyens commutateurs (18), aptes à relier ce condensateur à une borne de stimulation (16) du dispositif, elle-même reliée à une électrode de stimulation,
- des moyens de test de capture, aptes à déterminer, après délivrance d'une stimulation, si celle-ci a été efficace ou si, au contraire, il y a eu perte de capture,
- des moyens de réajustement de la tension de stimulation en fonction du résultat du test de capture, et
- des moyens de délivrance d'une contre-stimulation après test de capture si le résultat de ce test révèle une perte de capture,
dispositif **caractérisé en ce que** les moyens de délivrance d'une contre-stimulation incluent :
- des moyens condensateurs supplémentaires (32), et
- des seconds moyens commutateurs (34), aptes à relier ces moyens condensateurs supplémentaires à ladite borne de stimulation (16) du dispositif.

2. Le dispositif de la revendication 1, dans lequel :
- lesdits moyens condensateurs supplémentaires comprennent un condensateur spécifique (32), distinct dudit condensateur de sortie (12), et
- le circuit de charge (14) est également apte à charger ce condensateur spécifique à une tension (V2) de contre-stimulation supérieure à ladite tension de stimulation.

3. Le dispositif de la revendication 2, dans lequel il est prévu au moins deux étages de stimulation distincts, et dans lequel ledit condensateur spécifique (32) est un condensateur de sortie appartenant à un second étage autre que ledit étage de stimulation.

4. Le dispositif de la revendication 1, dans lequel :
- lesdits moyens condensateurs supplémentaires incluent un condensateur additionnel (32),
- le circuit de charge (14) est également apte à charger séparément ce condensateur additionnel, et
- les seconds moyens commutateurs sont des moyens (42, 46) aptes à relier en série le condensateur de sortie (12) et le condensateur additionnel (32) pendant la délivrance de la contre-stimulation.

5. Le dispositif de la revendication 4, dans lequel :
- une première armature du condensateur de sortie (12) est reliée à la fois à une première sortie (V1) du circuit de charge (14) et à un premier commutateur (18) relié par ailleurs sélectivement à une première borne de stimulation (16),
- la seconde armature du condensateur de sortie (12) est reliée à un deuxième commutateur (42) relié par ailleurs sélectivement à la masse,
- une première armature du condensateur additionnel (32) est reliée à la fois à une seconde sortie (V2) du circuit de charge (14) et à un troisième commutateur (18') relié par ailleurs sélectivement à une seconde borne de stimulation (16'), et
- un quatrième commutateur (46) relie sélectivement la seconde armature du condensateur de sortie (12) et la première armature du condensateur additionnel (32).

6. Le dispositif de la revendication 4, dans lequel il est prévu au moins deux étages de stimulation distincts, et dans lequel ledit condensateur additionnel (32) est un condensateur de sortie appartenant à un second étage autre que ledit étage de stimulation.

7. Le dispositif de la revendication 3 ou 4, dans lequel ledit étage de stimulation est un étage auriculaire et ledit second étage de stimulation est un étage ventriculaire droit, ou inversement.

8. Le dispositif de la revendication 3 ou 4, dans lequel ledit étage de stimulation est un étage auriculaire et ledit second étage de stimulation est un étage ventriculaire gauche, ou inversement.

9. Le dispositif de la revendication 3 ou 4, dans lequel ledit étage de stimulation est un étage ventriculaire droit et ledit second étage de stimulation est un étage ventriculaire gauche, ou inversement.

## Patentansprüche

1. Aktive implantierbare medizinische Vorrichtung, insbesondere ein Herzschrittmacher, Defibrillator und/oder Herzwandler oder Mehrstellen-Vorrichtung, aufweisend zumindest eine Stimulationsstufe mit schwacher Energie einer Herzkavität, wobei die Stimulationsstufe aufweist:
- einen Ausgangskondensator (12),
- eine Ladungsschaltung (14) des Kondensators mit vorbestimmter Stimulationsspannung (V1), nahe der Stimulationseffizienzstufe des Trägerpatienten der Vorrichtung,
- erste Umschaltmittel (18), geeignet zum Verbinden des Kondensators mit einem Stimulationsanschluss (16) der Vorrichtung, der seinerseits mit einer Stimulationselektrode verbunden ist,
- Verschlusstestmittel, geeignet zum Bestimmen, nach Abgabe einer Stimulation, ob diese wirksam gewesen ist oder ob es dagegen einen Verschlussverlust gegeben hat,
- Mittel zum Nachregeln der Stimulationsspannung in Abhängigkeit des Verschlusstestergebnisses, und
- Abgabemittel einer Gegenstimulation nach Verschlusstest, wenn das Ergebnis dieses Tests einen Verschlussverlust offenbart,
wobei die Vorrichtung **dadurch gekennzeichnet ist, dass** die Abgabemittel einer Gegenstimulation umfassen:
- zusätzliche Kondensatormittel (32) und
- zweite Umschaltmittel (34), geeignet zum Verbinden der zusätzlichen Kondensatormittel mit dem Stimulationsanschluss (16) der Vorrichtung.

2. Vorrichtung gemäß Anspruch 1, bei welcher:
- die zusätzlichen Kondensatormittel einen speziellen Kondensator (32) umfassen, unterschiedlich von dem Ausgangskondensator (12), und
- die Ladeschaltung (14) ebenfalls geeignet ist, diesen speziellen Kondensator zu laden mit einer Gegenstimulationsspannung (V2), die höher ist als die Stimulationsspannung.

3. Vorrichtung gemäß Anspruch 2, bei welcher zumindest zwei verschiedene Stimulationsstufen vorgesehen sind und bei welcher der spezielle Kondensator (32) ein Ausgangskondensator ist, welcher einer zweiten Stufe angehört, die eine andere ist als die Stimulationsstufe.

4. Vorrichtung gemäß Anspruch 1, bei welcher:
- die zusätzlichen Kondensatormittel einen zusätzlichen Kondensator (32) umfassen,
- die Ladeschaltung (14) ebenfalls geeignet ist, diesen zusätzlichen Kondensator separat zu laden, und
- die zweiten Umschaltmittel Mittel (42, 46) sind, die geeignet sind, den Ausgangskondensator (12) und den zusätzlichen Kondensator (32) in Serie zu verbinden während der Abgabe der Gegenstimulation.

5. Vorrichtung gemäß Anspruch 4, bei welcher:
- eine erste Platte des Ausgangskondensators (12) verbunden ist sowohl mit einem ersten Ausgang (V1) der Ladeschaltung (14) als auch mit einem ersten Umschalter (18), der des Weiteren selektiv mit einem ersten Stimulationsanschluss (16) verbunden ist,
- die zweite Platte des Ausgangskondensators (12) mit einem zweiten Umschalter (42) verbunden ist, der des Weiteren selektiv mit der Masse verbunden ist,
- eine erste Platte des zusätzlichen Kondensators (32) verbunden ist sowohl mit einem zweiten Ausgang (V2) der Ladeschaltung (14) als auch mit einem dritten Umschalter (18'), der des Weiteren selektiv verbunden ist mit einem zweiten Stimulationsanschluss (16'), und
- ein vierter Umschalter (46) selektiv die zweite Platte des Ausgangskondensators (12) und die erste Platte des zusätzlichen Kondensators (32) verbindet.

6. Vorrichtung gemäß Anspruch 4, bei welcher zumindest zwei verschiedene Stimulationsstufen vorgesehen sind und bei welcher der zusätzliche Kondensator (32) ein Ausgangskondensator ist, welcher einer zweiten Stufe angehört, die eine andere als die Stimulationsstufe ist.

7. Vorrichtung gemäß Anspruch 3 oder 4, bei welcher die Stimulationsstufe eine aurikuläre Stufe ist und die zweite Stimulationsstufe eine rechte ventrikuläre Stufe ist, oder umgekehrt.

8. Vorrichtung gemäß Anspruch 3 oder 4, bei welcher die Stimulationsstufe eine aurikuläre Stufe ist und die zweite Stimulationsstufe eine linke ventrikuläre Stufe ist, oder umgekehrt.

9. Vorrichtung gemäß Anspruch 3 oder 4, bei welcher die Stimulationsstufe eine rechte ventrikuläre Stufe ist und die zweite Stimulationsstufe eine linke ventrikuläre Stufe ist, oder umgekehrt.

## Claims

1. An active implantable medical device, in particular a cardiac pacemaker, defibrillator and/or cardioverter or a multisite device, comprising at least one stage for low energy stimulation of a cardiac cavity, said stimulation stage including:
- an output capacitor (12),
- a circuit (14) for charging said output capacitor to a predetermined stimulation voltage (V1), near to the stimulation effectiveness threshold of the patient carrying the device,
- first switch means (18), adapted to connect said capacitor to a stimulation output terminal (16) of the device, the terminal being connected to a stimulation electrode,
- means for performing a capture test, adapted to determine, after delivery of a stimulation, if this stimulation was effective or if, instead, there was a loss of capture,
- means for readjusting the stimulation voltage according to the result of the capture test; and
- means for delivering a backup stimulation after the capture test if the result of the capture test reveals a loss of capture,
said device being **characterized in that** said means for delivering a backup stimulation include:
- additional capacitor means (32), and
- second switch means (34), adapted to connect said additional capacitor means to said stimulation terminal (16) of the device.

2. The device of claim 1, wherein:
- said additional capacitor means include a specific capacitor (32), distinct from said output capacitor (12), and
- the charging circuit (14) is also adapted to charge said specific capacitor to a backup stimulation voltage (V2) that is higher than said stimulation voltage.

3. The device of claim 2, wherein there is provided at least two distinct stimulation stages, and wherein said specific capacitor (32) is an output capacitor included in a second stage other than said stimulation stage.

4. The device of claim 1, wherein:
- said additional capacitor means include an additional capacitor (32),
- the charging circuit (14) is-also adapted to separately charge said additional capacitor, and
- the second switch means are means (42, 46) adapted to connect the additional capacitor (32) in series with the output capacitor (12) during the delivery of the backup stimulation.

5. The device of claim 4, wherein:
- a first plate of the output capacitor (12) is connected both to a first output (V1) of the charging circuit (14) and to a first switch (18) which is further selectively connected to a first stimulation terminal (16),
- the second plate of the output capacitor (12) is connected to a second switch (42) which is further selectively connected to the ground potential,
- a first plate of the additional capacitor (32) is connected both to a second output (V2) of the charging circuit (14) and to a third switch (18') which is further selectively connected to a second stimulation terminal (16'), and
- a fourth switch (46) selectively connects the second plate of the output capacitor (12) and the first plate of the additional capacitor (32).

6. The device of claim 4, wherein there is provided at least two distinct stimulation stages, and wherein said additional capacitor (32) is an output capacitor included in a second stage other than said stimulation stage.

7. The device of claim 3 or 4, wherein said stimulation stage is an atrial stage and said second stimulation stage is a right ventricular stage, or conversely.

8. The device of claim 3 or 4, wherein said stimulation stage is an atrial stage and said second stimulation stage is a left ventricular stage, or conversely.

9. The device of claim 3 or 4, wherein said stimulation stage is a right ventricular stage and said second stimulation stage is a left ventricular stage, or conversely.
